# EUROPEAN PATENT SPECIFICATION

(11) **EP 3 143 975 B1**
(45) Date of publication and mention of the grant of the patent: **13.03.2019**
(21) Application number: 16186803.9
(22) Date of filing: 01.09.2016
(51) Int. Cl.: A61F 13/42, G01N 27/22

(54) **SMART NURSING CONSUMABLE, ROLLABLE SENSOR MODULE THEREOF AND MANUFACTURING METHOD THEREOF**
INTELLIGENTES VERBRAUCHSMATERIAL ZUM STILLEN, EINROLLBARES SENSORMODUL DAFÜR UND HERSTELLUNGSVERFAHREN DAFÜR
CONSOMMABLE DE SOINS INTELLIGENT, SON MODULE DE CAPTEUR ROULANT ET SON PROCEDE DE PRODUCTION

(30) Priority: 15.09.2015 CN 201510584755
(43) Date of publication of application: 22.03.2017
(73) Proprietor: Sinopulsar Technology Inc., Hsinchu City 31040 (TW)
(72) Inventor: WU, Tien Hsiang, 31040 Hsinchu City (TW)
(74) Representative: Reichert & Lindner Partnerschaft Patentanwälte

(56) References cited:
- WO-A2-2010/049827
- US-A1- 2008 284 608
- US-A1- 2014 148 772

## Description

### TECHNICAL FIELD OF THE INVENTION

The present invention relates to a diaper, and more particularly to a smart nursing consumable, a rollable sensor module of the smart nursing consumable, and a manufacturing method of the smart nursing consumable.

### BACKGROUND OF THE INVENTION

Conventionally, nursing consumables, such as gauze/swab, urinary pad, diaper, replacement sheets, etc., has no sensing functions, and therefore cannot notify the active condition of the nursing consumables or notify the care receiver as well as the caregivers to change the nursing consumables when become dirty. In addition, the conventional nursing consumables do not have physiological sensing functions, either. In generally, some care receiver such as infants, elderly with dementia or patients with limited mobility, etc. may not be able to self-control their excretions. Therefore, it is common among care receivers to wear diapers to lessen the burden of the caregivers.

However, moisture in diaper, PH of urine, and proteins or bacteria in feces tend to cause skin-related infections among diapered care receivers. Therefore, it is quite important for the caregivers to determine whether diapers are dirty and when to change dirty diapers. Conventionally, the determination of timing to change diapers is realized based on the caregivers actively and physically touching the diaper, thereby sensing whether the humidity inside the diaper is too high. Alternatively, caregivers would passively wait until the care receiver express discomfort. However, the aforementioned judgments depend primarily on personal experiences or feelings of the caregivers, and the caregivers may judge incorrectly when the care receivers give uncertain expressions.

As a result, smart diapers capable of sensing the presence of excretions has been developed, such as for example those of US 2008/0284608 A1, US 2014/0148772 A1 and WO 2010/049827 A2. Generally, a conventional smart diaper has a conductive tape formed by conductive printing on a waterproof layer (for example, PE materials for better skin sensation) of the smart diaper. The conductive tape is covered with a water-absorbing layer, and a transceiver assembly is used to sense the impedance changes of the conductive tape. When the water-absorbing layer absorbs certain amount of urine, some of the urine may permeate to the conductive tape and the impedance between the conductive tapes would change consequently. Therefore, once the transceiver assembly senses that the impedance between the conductive tapes has changed, it would notify that the water-absorbing layer has absorbed certain amount of urine and it is time to change a new smart diaper.

Conventionally, the waterproof layer is made smooth so that the conductive resins can be printed thereon and the connection between the tapes on the waterproof layer and the transceiver assembly can be realized more easily. However, because feces could not permeate to the conductive tape through the water-absorbing layer, the impedance between the conductive tapes would not change when the care receiver has excreted. Therefore, the determination diaper change for the care receiver still relies on the caregiver's attention. Moreover, convention smart diapers can only notify the caregivers when to change but not sense or analyze each urination of the care receivers. Additionally, some conventional smart diapers have a water-absorbing layer on a waterproof layer (made by PE materials for example) and a permeable layer on the water-absorbing layer; therefore, openings on the water-absorbing layer and the permeable layer are required to expose the connecting terminals of the tapes or wires on the waterproof layer, so that the connecting terminals can be electrically connected to the transceiver assembly. The openings are covered by a release paper when the smart diapers are manufactured. When the smart diapers are used, users must first remove the release paper to expose the openings and then electrically connect the transceiver assembly to the connecting terminals of the tapes or wires.

However, having the openings and the release paper in the conventional smart diapers, the cost of production is increased. In addition, the conventional smart diapers may inconvenience for that the users must remove the release paper before using the smart diapers. Further, the release paper may be accidentally detached from unused smart diapers. Furthermore, because the conventional smart diapers have the water-absorbing layer and the permeable layer formed on the waterproof layer, the wires tend to exhibit poor sensibility; namely, the wires can only sense urine but not feces.

### SUMMARY OF THE INVENTION

One objective of the present invention is to provide a smart nursing consumable with improved sensibility.

Another objective of the present invention is to provide a rollable sensor module of the aforementioned smart nursing consumable.

Still another objective of the present invention is to provide a manufacturing method of the smart nursing consumable, thereby improving the production efficiency of the smart nursing consumable.

The present invention provides a smart nursing consumable, which includes an absorbent body and a sensor module disposed on the absorbent body. The sensor module includes a first film sheet, at least one wire, a second film sheet and a conductive pad. The first film sheet is disposed on the absorbent body. The at least one wire is disposed on the first film sheet. The second film sheet is disposed on the first film sheet and covers the at least one wire. The conductive pad is connected to the at least one wire without passing through the second film sheet. Both of the first film sheet and the second film sheet include a plurality of pores.

In one embodiment, the conductive pad is disposed on the second film sheet and connected to the at least one wire through a portion of the plurality of pores of the second film sheet.

In one embodiment, the conductive pad is disposed between the first film sheet and the second film sheet.

In one embodiment, the first film sheet and the second film sheet are non-woven fabric or adhesive-bonded fabric.

In one embodiment, a material of the conductive pad is selected from at least one element in a group consisting of conductive plastic gum, conductive gel, conductive plastic, conductive ink, conductive paint and carbon film.

In one embodiment, a quantity of the at least one wire may be two or more than two.

In one embodiment, the smart nursing consumable comprises smart nursing pants, a smart diaper, a smart nursing pad, a smart sanitary napkin or smart gauze.

The present invention also provides a rollable sensor module of a smart nursing consumable according to any of claims 8 or 9. The rollable sensor module includes a first film sheet, at least one wire and a second film sheet. The at least one wire is disposed on the first film sheet. The second film sheet is disposed on the first film sheet and covers the at least one wire. Both of the first film sheet and the second film sheet includes a plurality of pores.

In one embodiment, the aforementioned rollable sensor module further includes a conductive pad. The conductive pad is disposed between the first film sheet and the second film sheet and is connected to the at least one wire.

The aforementioned rollable sensor module further includes a conductive pad. The conductive pad is disposed between the first film sheet and the second film sheet and connected to the at least one wire through a portion of the plurality of pores of the second film sheet.

The present invention further provides a manufacturing method of a smart nursing consumable. The method includes: providing an absorbent body; and disposing a portion of the aforementioned rollable sensor module on the absorbent body.

In summary, because the sensor module is disposed above the absorbent body, the smart nursing consumable of the present invention has improved sensibility without affecting urine permeability and skin sensation. In addition, because the wires are covered by the second film sheet, comfort of the user and connectivity of the wires would not be affected, as compared with conventional diapers. Further, as compared with smart nursing consumables of the prior art, the smart nursing consumable of the present invention does not require the use of release papers; and consequently, not only the cost for the release paper is saved, but also the issue of accidental detachment of the release paper is avoided. Furthermore, the smart nursing consumable of the present invention has improved usability as removal of the release paper before using the smart nursing consumable is no longer required. Furthermore, because the smart nursing consumable of the present invention is manufactured by cutting off and disposing the rollable sensor module on the absorbent body, the manufacturing method of the smart nursing consumable of the present invention has improved production efficiency.

### BRIEF DESCRIPTION OF THE DRAWINGS

The present disclosure will become more readily apparent to those ordinarily skilled in the art after reviewing the following detailed description and accompanying drawings, in which:
FIG. 1 is a perspective schematic view of a smart nursing consumable in accordance with an embodiment of the present invention;
FIG. 2 is a partial cross-sectional schematic view of an absorbent body clamped by a transceiver assembly in accordance with an embodiment of the present invention;
FIG. 3 is a partial cross-sectional schematic view of a smart nursing consumable in accordance with another embodiment of the present invention;
FIGS. 4A and 4B are perspective and cross-sectional schematic views of a rollable sensor module in accordance with an embodiment of the present invention, respectively;
FIG. 5 is a cross-sectional schematic view of a rollable sensor module in accordance with another embodiment of the present invention;
FIG. 6 is a cross-sectional schematic view of a rollable sensor module in accordance with yet another embodiment of the present invention; and
FIGS. 7A-7C are schematic views for illustrating a method of manufacturing a smart nursing consumable in accordance with an embodiment of the present invention.

### DETAILED DESCRIPTION OF PREFERRED EMBODIMENTS

The present disclosure will now be described more specifically with reference to the following embodiments. It is to be noted that the following descriptions of preferred embodiments of this disclosure are presented herein for purpose of illustration and description only. It is not intended to be exhaustive or to be limited to the precise form disclosed.

The present invention provides a smart nursing consumable for a care receiver and for detecting and collecting excretions of the care receiver. The smart nursing consumable of the present invention may be implemented as smart nursing pants (such as smart diapers), smart nursing pads, smart sanitary napkins for menstruation or smart gauze for covering wounds; and the present invention is not limited thereto. In addition, the smart nursing consumable of the present invention may be further implemented as mats or packaging materials for collecting excretions of the care receiver. The smart nursing consumable of the present invention will be exemplarily implemented as smart nursing pants, and the structures and functions thereof will be described in detail as follow.

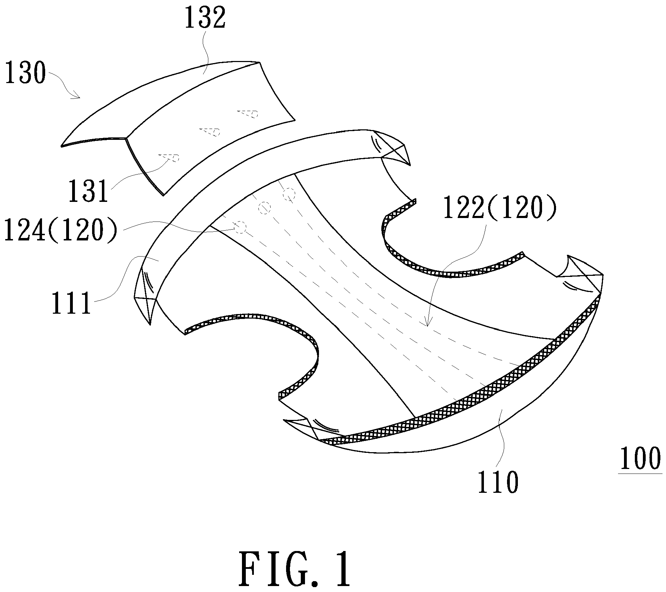

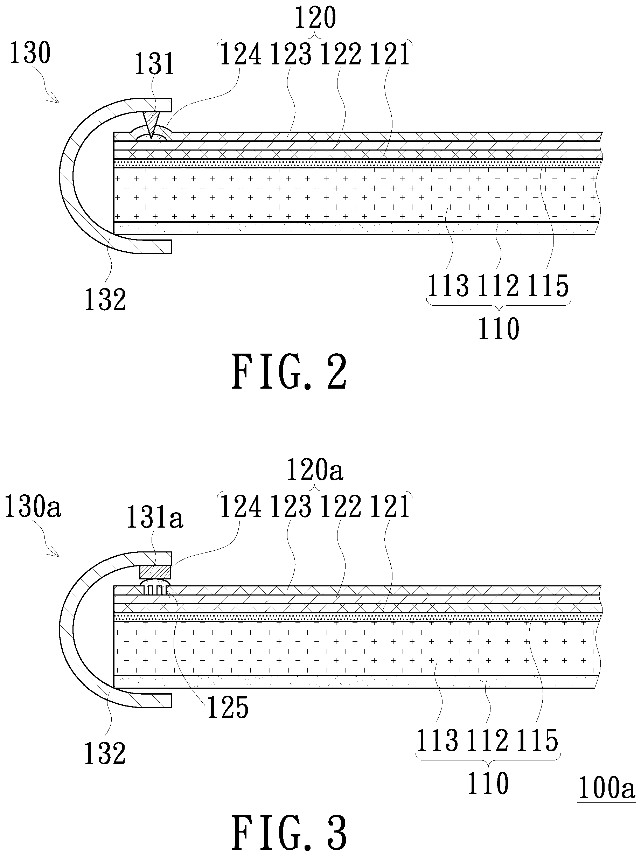

FIG. 1 is a perspective schematic view of a smart nursing consumable in accordance with an embodiment of the present invention. FIG. 2 is a partial cross-sectional schematic view of an absorbent body clamped by a transceiver assembly in accordance with an embodiment of the present invention. Please refer to FIGS. 1 and 2. The smart nursing consumable 100 of the present embodiment includes an absorbent body 110 and a sensor module 120 disposed on the absorbent body 110. The sensor module 120 includes a first film sheet 121, wires 122, a second film sheet 123 and a conductive pad 124. Specifically, the first film sheet 121 is disposed on the absorbent body 110; the wires 122 are disposed on the first film sheet 121; and the second film sheet 123 is disposed on the first film sheet 121 and covers the wires 122. Both of the first film sheet 121 and the second film sheet 123 include a plurality of pores (not shown), so that the first film sheet 121 and the second film sheet 123 can function as a permeable layer.

In the present embodiment, the wires 122 and the conductive pad 124 as illustrated by dotted lines in FIG. 1 are disposed between the first film sheet 121 and the second film sheet 123. The first film sheet 121 and the second film sheet 123 may be non-woven fabric or adhesive-bonded fabric with pores or other permeable materials with pores. The quantity of the wire 122 corresponds to that of the conductive pad 124. In the present embodiment, the sensor module 120 is exemplified by including three wires 122 and three conductive pads 124 as shown in FIG. 1. However, the quantities of the wire 122 and the conductive pad 124 are not limited in the present invention. In other words, the sensor module 120 may include two wires 122 and two conductive pads 124 in another embodiment; or, the sensor module 120 may include more than three wires 122 and the same number of conductive pads 124 in yet another embodiment. It is to be understood that the sensing area of the sensor module 120 increases with the quantities of the wire 122 and the conductive pad 124, thereby improving the overall sensibility of the smart nursing consumable 100. The wires 122 may metal wires or metal films. The material of the conductive pad 124 may be conductive plastic gum, conductive plastic, conductive ink, conductive paint or other liquid conductive material. In one embodiment, the material of the conductive pad 124 is carbon film. In addition, it is to be understood that the conductive pad 124 may contain two or more of the aforementioned materials. The absorbent body 110 includes a waterproof layer 112 and a water-absorbing layer 113 disposed on the waterproof layer 112. The first film sheet 121 of the sensor module 120 is disposed on the water-absorbing layer 113. In addition, the absorption body 110 may further include a water draining layer 115. The water draining layer 115 is disposed above the water-absorbing layer 113, and the sensor module 120 is disposed on the water draining layer 115.

In the present embodiment, the smart nursing consumable 100 can be coupled to the transceiver assembly 130. Specifically, the transceiver assembly 130 is coupled to the sensor module 120. More specifically, the transceiver assembly 130 includes a connecting member 131 for connecting to the conductive pad 124 of the sensor module 120. When the transceiver assembly 130 is coupled to the sensor module 120, the connecting member 131 passes through the second film sheet 123 to connect to the conductive pad 124, thereby electrically connecting to the wires 122. The transceiver assembly 130 may further include a clamp body 132, in which the connecting member 131 is disposed. The clamp body 134 may be a flexible circuit board or a rigid circuit board, and the present invention is not limited thereto. The connecting member 131 may be a pin connecting member, so that the connecting member 131 can be inserted into the absorbent body 110. The transceiver assembly 130 may further include an impedance sensor (not shown). The impedance sensor is electrically connected to the connecting member 131 and configured to sense the impedance value between the wires 122 so as to obtain a plurality of time-dependent impedance values and a time-dependent impedance change. A processing element (not shown) is employed and configured to analyze the time-dependent change in impedance values between the wires 122, so as to determine and predict the occurrence of urination and excretion of the care receiver and to issue an alarm timely. The processing element may be disposed in the transceiver assembly 130 or in a monitoring module (not shown) that is signally connected to the transceiver assembly 130. The conductive pad 124 is disposed close to a belt 111 of the absorbent body 110 and the clamp body 132 clamps the belt 111; therefore, the connection between the connecting member 131 and the conductive pad 124 is realized.

In the present embodiment, when the transceiver assembly 130 is coupled to the absorbent body 110, the connecting member 131 passes through the second film sheet 123 to connect to the conductive pad 124, so that the transceiver assembly 130 can be electrically connected to the wire 122 through the conductive pad 124. As compared to smart diapers in the prior art, the smart nursing consumable 100 of the present embodiment does not have any openings; and therefore, no release paper for covering openings is required. As a result, not only the cost for the release paper is saved but also the issue of accidental detachment of the release paper is avoided. Further, the smart nursing consumable 100 of the present embodiment has improved usability as removal of the release paper before using the smart nursing consumable 100 is no longer required.

In addition, being disposed above the absorbent body 110, the sensor module 120 has improved sensibility. Specifically, in each urination, the urine would pass through the wires 122 and eventually be absorbed by the water-absorbing layer 113. When the urine permeates to the wires 122, the impedance value between the wires 122 would exhibit a significant change; then, the impedance value change is narrowed when the urine permeated to the wires 122 is gradually absorbed by the water-absorbing layer 113; and eventually the impedance value returns to the original value when the urine is completely absorbed by the water-absorbing layer 113. As a result, in each urination, the impedance sensor can sense a time-dependent waveform-like impedance change. Through the waveform-like impedance change, the time and duration of each urination are recorded and analyzed; and consequently, the number of times and frequency of urination, as well as the amount of urine, etc. are obtained. As a result, the detection and determination of the health condition of the care receiver, such as urination frequency or urinary tract infections, are facilitated. In addition, when the care receiver excretes, the feces would pass through the pores of the second film sheet 123 and stick to the wires 122 or the areas between the wires 122; and therefore, the impedance value changed due to the excrements would not return back to the original value. As a result, every time when an unrecoverable impedance value is observed, excretion of the care receiver can be determined. Further, according to the quantity of the wires 122 having short circuit, areas of the smart nursing consumable 100 wetted by urine or other liquids can be determined. Further, the smart nursing consumable 100 of the present invention may be implemented with some specific technical features to measure reactance or resistance by frequency or to analyze PH values, thereby facilitating the sensing and determination of the care receiver's physiological conditions. Therefore, it is to be understood that the present invention is different from the prior art, in which only the threshold value of resistance is measured.

In the smart nursing consumable 100 of the present embodiment, because the sensor module 120 is disposed above the absorbent body 110, the urination or excretion of the care receiver can be detected and determined according to the waveform of the sensed signal, thereby facilitating the determination and prediction of the care receiver's conditions. In addition, to provide comfort without any foreign body sensation and to prevent the sensor module 120 from blocking liquid permeation, the present invention employs the wires 122 instead of the conductive tapes used in the prior art. In a preferred embodiment, the width of the wire 122 ranges from 0.05 millimeter (mm) to 1 mm. Having such widths, the wires 122 tend to fall into the pores of the first film sheet 121 or the second film sheet 123, leading to poor connection between the wires 122 and the connecting member 131 of the clamp body 132; therefore, the conductive pad 124 is employed to avoid the occurrence of the aforementioned situation and the resulted error signal. In addition, by using the first film sheet 121 and the second film sheet 123 to wrap around the wires 122, the wires 122 are prevented from directly contact with the care receiver's skin and the water draining layer 115, thus avoiding the error signal. In addition, the wire 122 may be made of alloy to avoid the wire breakage. In one embodiment, the wire 122 is made of tin-copper alloy, but the present invention is not limited thereto.

When being applied to smart gauze for covering wounds, the smart nursing consumable 100 of the present invention may be used to sense and analyze whether the wound is bleeding and whether the breeding is excessive.

FIG. 3 is a partial cross-sectional schematic view of a smart nursing consumable in accordance with another embodiment of the present invention. As shown in FIG. 3, the structures and advantages of the smart nursing consumable 100a of the present embodiment are similar to those of the smart nursing consumable 100 in the embodiment of FIG. 2; therefore, no redundant detail is to be given herein and only the difference in structures between the two will be described in the followings. In the present embodiment, the conductive pad 124 of the sensor module 120a is disposed on the second film sheet 123 and connected to the wires 122 through a portion of the pores 125 of the second film sheet 123. In addition, the connecting member 131a of the transceiver assembly 130a can be connected to the conductive pad 124 without passing through the second film sheet 123; therefore, there is no need to design the connecting member 131a as a pin connecting member. It is to be noted that the pores 125 illustrated in FIG. 3 are for an exemplary purpose only; in fact, the second film sheet 123 is entirely formed with the pores 125.

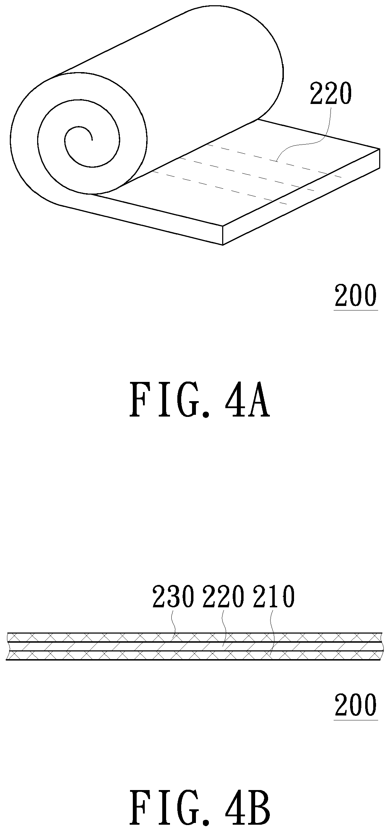

FIGS. 4A and 4B are perspective and cross-sectional schematic views of a rollable sensor module in accordance with an embodiment of the present invention, respectively. The rollable sensor module 200 of the present embodiment shown in FIGS. 4A and 4B can be applied to smart nursing consumables, leather or any products requiring moisture or water permeation control; however, it is to be understood that the fields of application of the rollable sensor module 200 is not limited in the present invention. As shown in FIGS. 4A and 4B, the rollable sensor module 200 includes a first film sheet 210, wires 220 and a second film sheet 230. Specifically, the wires 220 are disposed on the first film sheet 210; and the second film sheet 230 is disposed on the first film sheet 210 and covers the wires 220. Both of the first film sheet 210 and the second film sheet 230 include a plurality of pores (not shown), so that the first film sheet 210 and the second film sheet 230 can function as a permeable layer. The first film sheet 210 and the second film sheet 230 may be non-woven fabric, adhesive-bonded fabric, gauze fabric or other permeable materials; and it is to be understood that the materials of the first film sheet 210 and the second film sheet 230 can be selected based on the product to which the rollable sensor module 200 applies. The wires 220 may be metal wires or metal films, and the present invention is not limited thereto.

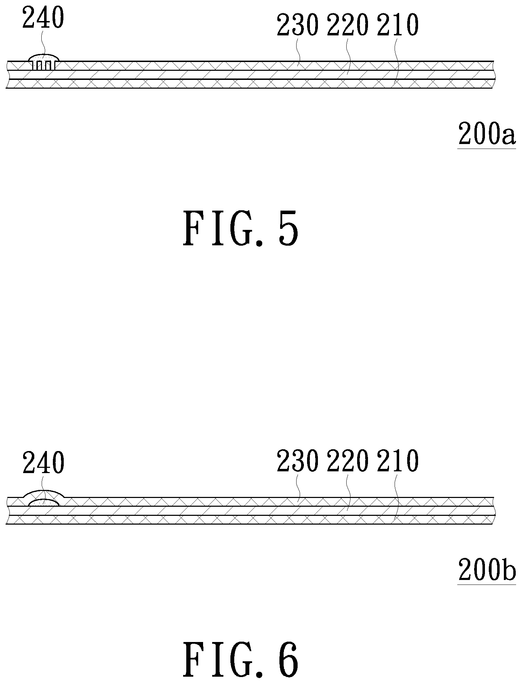

FIG. 5 is a cross-sectional schematic view of a rollable sensor module in accordance with another embodiment of the present invention. FIG. 6 is a cross-sectional schematic view of a rollable sensor module in accordance with yet another embodiment of the present invention. As shown in FIGS. 5 and 6, as compared with the rollable sensor module 200 in the embodiment of FIG. 4B, rollable sensor modules 200a, 200b of the present embodiments further include the conductive pad 240. Specifically, the conductive pad 240 of the rollable sensor module 200a is disposed on the second film sheet 230; and the conductive pad 240 of the rollable sensor module 200b is disposed between the first film sheet 210 and the second film sheet 230. The material of the conductive pad 240 may be conductive plastic gum, conductive plastic, or other conductive material. In one embodiment, the material of the conductive pad 240 is carbon film. In one embodiment, the conductive pad 240 may be formed by printing or dispensing; however, the means for the formation of the conductive pad 240 is not limited in the present invention. In addition, the quantity of the conductive pad 240 may be multiple, and the plurality of conductive pads 240 can be distributed throughout the rollable sensor modules 200a, 200b in response to the design needs.

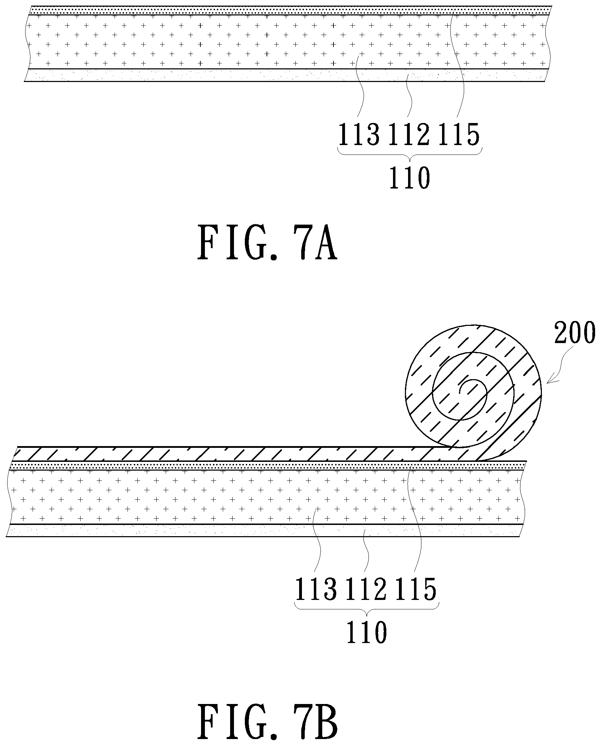

FIGS. 7A-7C are schematic views for illustrating a method of manufacturing a smart nursing consumable in accordance with an embodiment of the present invention. The method of manufacturing the smart nursing consumable of the present embodiment includes the following steps. First, as shown in FIG 7A, the absorbent body 110 is provided. Specifically, the absorbent body 110 includes the waterproof layer 112 and the water-absorbing layer 113 disposed on the waterproof layer 112. The absorption body 110 may further include the water draining layer 115. The water draining layer 115 is disposed above the water-absorbing layer 113, and the sensor module 120 is disposed on the water draining layer 115.

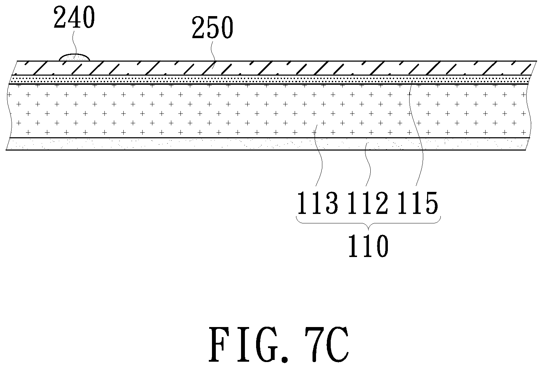

Next, as shown in FIG. 7B, a portion of the rollable sensor module 200 is disposed on the absorbent body 110 to form a sensor module 250 (show in FIG. 7C). Specifically, the sensor module 250 on the absorbent body 110 is formed by disposing the rollable sensor module 200 on the water draining layer 115 of the absorbent body 110 and then cutting off the rollable sensor module 200.

Next, as shown in FIG. 7C, the conductive pad 240 is formed on the sensor module 250 as the rollable sensor module 200 does not have a conductive pad. The conductive pad 240 is electrically connected to the transceiver assembly. The material of the conductive pad 240 may be conductive plastic gum, conductive plastic, or other conductive material. In one embodiment, the material of the conductive pad 240 is carbon film. In one embodiment, the conductive pad 240 may be formed by printing or dispensing; however, the means for the formation of the conductive pad 240 is not limited in the present invention.

In other embodiments, where the rollable sensor modules 200a, 200b equipped with the conductive pad 240 are used for manufacturing the sensor module 250, the step illustrated in FIG. 7C for forming the conductive pad 240 can be omitted. Namely, the sensor module 250 can be manufactured by directly cutting off the rollable sensor modules 200a, 200b on the absorbent body 110.

Because the sensor module 250 is manufactured by cutting off and disposing the rollable sensor modules 200, 200a or 200b on the absorbent body 110, the method of manufacturing a smart nursing consumable of the present invention has improved production efficiency.

In summary, because the sensor module is disposed above the absorbent body, the smart nursing consumable of the present invention has improved sensibility. In addition, because the wires are covered by the second film sheet, comfort of the use and connectivity of the wires would not be affected. Further, as compared with the smart nursing consumables of the prior art, the smart nursing consumable of the present invention does not require the use of release papers; and consequently, not only the cost for the release paper is saved, but also the issue of accidental detachment of the release paper is avoided. Furthermore, the smart nursing consumable of the present invention has improved usability as removal of the release paper before using the smart nursing consumable is no longer required. Furthermore, because the smart nursing consumable of the present invention is manufactured by cutting off and disposing the rollable sensor module on the absorbent body, the manufacturing method of the smart nursing consumable of the present invention has improved production efficiency.

While the disclosure has been described in terms of what is presently considered to be the most practical and preferred embodiments, it is to be understood that it is intended to cover various modifications and similar arrangements included within the scope of the appended claims which are to be accorded with the broadest interpretation so as to encompass all such modifications and similar structures.

## Claims

1. A smart nursing consumable (100, 100a) coupled to a transceiver assembly (130, 130a), comprising:
an absorbent body (110);
**characterized by**
a sensor module (120, 120a, 200, 200a, 200b), disposed on the absorbent body (110), the sensor module (120, 120a) comprising:
a first film sheet (121, 210), disposed on the absorbent body (110);
at least one wire (122, 220), disposed on the first film sheet (121, 210);
a second film sheet (123, 230), disposed on the first film sheet (121, 210) and covering the at least one wire (122, 220); and
a conductive pad (124, 240), connected to the at least one wire (122, 220) without passing through the second film sheet (123, 230),
wherein both of the first film sheet (121, 210) and the second film sheet (123, 230) comprise a plurality of pores (125).

2. The smart nursing consumable (100, 100a) according to claim 1, wherein the conductive pad (124, 240) is disposed on the second film sheet (123, 230) and connected to the at least one wire (122, 220) through a portion of the plurality of pores (125) of the second film sheet (123, 230).

3. The smart nursing consumable (100, 100a) according to anyone of the preceding claims, wherein the conductive pad (124, 240) is disposed between the first film sheet (121, 210) and the second film sheet (123, 230).

4. The smart nursing consumable (100, 100a) according to anyone of the preceding claims, wherein the first film sheet (121, 210) and the second film sheet (123, 230) are non-woven fabric or adhesive-bonded fabric.

5. The smart nursing consumable (100, 100a) according to anyone of the preceding claims, wherein a material of the conductive pad (124, 240) is selected from at least one element in a group consisting of conductive plastic gum, conductive gel, conductive plastic, conductive ink, conductive paint and carbon film.

6. The smart nursing consumable (100, 100a) according to anyone of the preceding claims, wherein a quantity of the at least one wire (122, 220) is two or more than two.

7. The smart nursing consumable (100, 100a) according to anyone of the preceding claims, wherein the smart nursing consumable (100, 100a) comprises smart nursing pants, a smart diaper, a smart nursing pad, a smart sanitary napkin or smart gauze.

8. A rollable sensor module (200, 200a, 200b) of a smart nursing consumable (100, 100a) coupled to a transceiver assembly (130, 130a), comprising:
a first film sheet (121, 210);
at least one wire (122, 220), disposed on the first film sheet (121, 210);
a second film sheet (123, 230), disposed on the first film sheet (121, 210) and covering the at least one wire (122, 220), and
a conductive pad (124, 240), disposed between the first film sheet (121) and the second film sheet (123, 230) and connected to the at least one wire (122, 220),
wherein both of the first film sheet (121, 210) and the second film sheet (123, 230) comprise a plurality of pores (125).

9. The rollable sensor module (200, 200a, 200b) according to claim 8, wherein the conductive pad (124, 240) is disposed on the second film sheet (123, 230) and connected to the at least one wire (122, 220) through a portion of the plurality of pores (125) of the second film sheet (123, 230).

10. A manufacturing method of a smart nursing consumable (100, 100a), comprising:
providing an absorbent body (110); and
disposing a portion of the rollable sensor module (200, 200a, 200b) according to anyone of the claims 8 to 10 on the absorbent body (110).

## Patentansprüche

1. Intelligentes Pflegeverbrauchsmaterial (100, 100a), das mit einer Sender-Empfänger-Anordnung (130, 130a) gekoppelt ist, umfassend:
einen absorbierenden Körper (110);
**gekennzeichnet durch**
ein Sensormodul (120, 120a, 200, 200a, 200b), das auf dem absorbierenden Körper (110) angeordnet ist, wobei das Sensormodul (120, 120a) umfasst:
ein erstes Filmblatt (121, 210), das auf dem absorbierenden Körper (110) angeordnet ist;
mindestens einen Draht (122, 220), der auf dem ersten Filmblatt (121, 210) angeordnet ist;
ein zweites Filmblatt (123, 230), das auf dem ersten Filmblatt (121, 210) angeordnet ist und den mindestens einen Draht (122, 220) abdeckt; und
ein leitfähiges Pad (124, 240), das mit dem mindestens einen Draht (122, 220) verbunden ist, ohne durch das zweite Filmblatt (123, 230) zu verlaufen,
wobei sowohl das erste Filmblatt (121, 210) als auch das zweite Filmblatt (123, 230) eine Vielzahl von Poren (125) umfassen.

2. Intelligentes Pflegeverbrauchsmaterial (100, 100a) nach Anspruch 1, wobei das leitfähige Pad (124, 240) auf dem zweiten Filmblatt (123, 230) angeordnet und mit dem mindestens einen Draht (122, 220) durch einen Abschnitt der Vielzahl von Poren (125) des zweiten Filmblatts (123, 230) verbunden ist.

3. Intelligentes Pflegeverbrauchsmaterial (100, 100a) nach einem der vorherigen Ansprüche, wobei das leitfähige Pad (124, 240) zwischen dem ersten Filmblatt (121, 210) und dem zweiten Filmblatt (123, 230) angeordnet ist.

4. Intelligentes Pflegeverbrauchsmaterial (100, 100a) nach einem der vorherigen Ansprüche, wobei das erste Filmblatt (121, 210) und das zweite Filmblatt (123, 230) Vliesstoff oder klebstoffgebundener Stoff sind.

5. Intelligentes Pflegeverbrauchsmaterial (100, 100a) nach einem der vorherigen Ansprüche, wobei ein Material des leitfähigen Pads (124, 240) aus mindestens einem Element einer Gruppe ausgewählt ist, die aus leitfähigem Kunststoffgummi, leitfähigem Gel, leitfähigem Kunststoff, leitfähiger Tinte, leitfähiger Farbe und Kohlenstofffolie besteht.

6. Intelligentes Pflegeverbrauchsmaterial (100, 100a) nach einem der vorherigen Ansprüche, wobei eine Anzahl des mindestens einen Drahtes (122, 220) zwei oder mehr als zwei beträgt.

7. Intelligentes Pflegeverbrauchsmaterial (100, 100a) nach einem der vorherigen Ansprüche, wobei das intelligente Pflegeverbrauchsmaterial (100, 100a) intelligente Pflegehosen, eine intelligente Windel, ein intelligentes Pflegepad, eine intelligente Hygienebinde oder eine intelligente Gaze umfasst.

8. Rollbares Sensormodul (200, 200a, 200b) eines intelligenten Pflegeverbrauchsmaterials (100, 100a), das mit einer Sender-EmpfängerAnordnung (130, 130a) gekoppelt ist, umfassend:
ein erstes Filmblatt (121, 210);
mindestens einen Draht (122, 220), der auf dem ersten Filmblatt (121, 210) angeordnet ist;
ein zweites Filmblatt (123, 230), das auf dem ersten Filmblatt (121, 210) angeordnet ist und den mindestens einen Draht (122, 220) abdeckt, und
ein leitfähiges Pad (124, 240), das zwischen dem ersten Filmblatt (121, 210) und dem zweiten Filmblatt (123, 230) angeordnet und mit dem mindestens einen Draht (122, 220) verbunden ist,
wobei sowohl das erste Filmblatt (121, 210) als auch das zweite Filmblatt (123, 230) eine Vielzahl von Poren (125) umfassen.

9. Rollbares Sensormodul (200, 200a, 200b) nach Anspruch 8, wobei das leitfähige Pad (124, 240) auf dem zweiten Filmblatt (123, 230) angeordnet und durch einen Abschnitt der Vielzahl von Poren (125) des zweiten Filmblatts (123, 230) mit dem mindestens einen Draht (122, 220) verbunden ist.

10. Herstellungsverfahren für ein intelligentes Pflegeverbrauchsmaterial (100, 100a), umfassend:
Bereitstellen eines absorbierenden Körpers (110); und
Anordnen eines Abschnitts des rollbaren Sensormoduls (200, 200a, 200b) nach einem der Ansprüche 8 bis 10 auf dem absorbierenden Körper (110).

## Revendications

1. Consommable d'allaitement intelligent (100, 100a) couplé à un ensemble émetteur-récepteur (130, 130a), comprenant:
un corps absorbant (110);
**caractérisé par**
un module capteur (120, 120a, 200, 200a, 200b), disposé sur le corps absorbant (110), le module capteur (120, 120a) comprenant:
une première feuille de film (121, 210), disposée sur le corps absorbant (110);
au moins un fil (122, 220), disposé sur la première feuille de film (121, 210);
une deuxième feuille de film (123, 230), disposée sur la première feuille de film (121, 210) et recouvrant le au moins un fil (122, 220); et
un tampon conducteur (124, 240), connecté à l'au moins un fil (122, 220) sans passer à travers la seconde feuille de film (123, 230),
dans laquelle la première feuille de film (121, 210) et la seconde feuille de film (123, 230) comprennent toutes deux une pluralité de pores (125).

2. Consommable d'allaitement intelligent (100, 100a) selon la revendication 1, dans lequel le tampon conducteur (124, 240) est disposé sur la deuxième feuille de film (123, 230) et relié au moins un fil (122, 220) par une partie de la pluralité de pores (125) de la deuxième feuille de film (123, 230).

3. Consommable d'allaitement intelligent (100, 100a) selon l'une quelconque des revendications précédentes, dans lequel le tampon conducteur (124, 240) est disposé entre la première feuille de film (121, 210) et la seconde feuille de film (123, 230).

4. Consommable d'allaitement intelligent (100, 100a) selon l'une quelconque des revendications précédentes, dans lequel la première feuille de film (121, 210) et la seconde feuille de film (123, 230) sont un tissu non tissé ou un tissu collé.

5. Consommable d'allaitement intelligent (100, 100a) selon l'une quelconque des revendications précédentes, dans lequel un matériau de le tampon conducteur (124, 240) est choisi parmi au moins un élément d'un groupe constitué par une gomme plastique conductrice, un gel conducteur, un plastique conducteur, une encre conductrice, une peinture conductrice et un film de carbone.

6. Consommable d'allaitement intelligent (100, 100a) selon l'une quelconque des revendications précédentes, dans lequel une quantité d'au moins un fil (122, 220) est deux ou plus de deux.

7. Consommable d'allaitement intelligent (100, 100a) selon l'une quelconque des revendications précédentes, dans lequel le consommable d'allaitement intelligent (100, 100a) comprend un pantalon d'allaitement intelligent, une couche-culotte intelligente, une serviette hygiénique intelligente ou une gaze intelligente.

8. Module capteur enroulable (200, 200a, 200b) d'un consommable d'allaitement intelligent (100, 100a) couplé à un ensemble émetteur-récepteur (130, 130a), comprenant:
une première feuille de film (121, 210);
au moins un fil (122, 220), disposé sur la première feuille de film (121, 210);
une deuxième feuille de film (123, 230), disposée sur la première feuille de film (121, 210) et recouvrant d'au moins un fil (122, 220), et
un tampon conducteur (124, 240), disposé entre la première feuille de film (121) et la deuxième feuille de film (123, 230) et relié à l'au moins un fil (122, 220),
dans lequel la première feuille de film (121, 210) et la seconde feuille de film (123, 230) comprennent toutes deux une pluralité de pores (125).

9. Le module capteur enroulable (200, 200a, 200b) selon la revendication 8, dans lequel le tampon conducteur (124, 240) est disposé sur la deuxième feuille de film (123, 230) et relié au moins un fil (122, 220) par une partie de la pluralité de pores (125) de la deuxième feuille de film (123, 230).

10. Procédé de fabrication d'un consommable d'allaitement intelligent (100, 100a), comprenant:
la fourniture d'un corps absorbant (110); et
la disposition d'une partie du module de capteur enroulable (200, 200a, 200b) selon l'une quelconque des revendications 8 à 10 sur le corps absorbant (110).
